# EUROPEAN PATENT SPECIFICATION

(11) **EP 0 667 128 B1**
(45) Date of publication and mention of the grant of the patent: **27.05.1998**
(21) Application number: 95101332.5
(22) Date of filing: 24.08.1992
(51) Int. Cl.: A61N 7/00, A61F 2/46

(54) **Improved tool for removal of plastics material**
Werkzeug zur Entfernung von Kunststoff
Instrument amélioré pour enlever de la matière plastique

(30) Priority: 24.08.1991 GB 9118307
(43) Date of publication of application: 16.08.1995
(62) Divisional of application: 92917959.6
(73) Proprietor: YOUNG, Michael John Radley, Ashburton, South Devon TQ13 7JX (GB); BRADNOCK, Brian Robert Denis Peter, Hemel Hempstead, HP3 0PD (GB)
(72) Inventor: YOUNG, Michael John Radley, Ashburton, South Devon TQ13 7JX (GB); BRADNOCK, Brian Robert Denis Peter, Hemel Hempstead, HP3 0PD (GB)
(74) Representative: Gregory, Timothy Mark

(56) References cited:
- WO-A- /10423
- GB-A- 2 229 660
- US-A- 4 248 232

## Description

The present invention relates to an improved tool for use in removal of plastics material. A tool of this general type is disclosed in our GB 2229660, but further improvements have been discovered. The tool is particularly, but not exclusively, useful in removing plastics cement from such bores in bones as may be used in hip, or other joint, replacements (hereinafter referred to, for convenience, as hip joint replacements).

In a hip joint replacement operation, a metal implant is provided with a long projection which is inserted into a hole drilled in the medulla of the femur and is held firmly in place by means of a plastics cement. On average, such replacements can be expected to last five to ten years. However, due to repetitive shearing forces during daily use, either the bone/cement interface or the cement/metal interface may weaken and the implant will become loose, requiring revision. Sometimes, the metal of the hip replacement may fracture or the plastics components of it may wear out. In these cases, revision is necessary although in most cases the bone/cement interface usually remains quite strong.

In order to revise any loose or damaged implant, all or most of the plastics cement must be removed before inserting a new prosthesis and re-cementing. Removal of the old cement presents a number of difficulties. It is time-consuming and may cause fracturing of the bone. It involves the careful and tedious use of hand tools such as hammers and cement cutting chisels. High speed burrs have been used, but they frequently perforate the bone and make re-cementing more difficult and not so effective.

It is known from the above GB 2229660 to provide a tool for melting ultrasonically the cement in a bore from which a prosthesis has been removed. Such a tool has a plurality of holes through which cement melted on a forward working face passes to set on a rearward face to be removed on retraction of the tool. However, there are occassions when larger amounts of cement could be removed and in this case, it may be advisable to separate the cement into segments which can be more easily removed. This may be done with providing the work surface with radially extending fins.

It is an object of the present invention to provide an improved tool for removal of plastics material such as cement from a bore, particularly one in a bone, which overcomes the above disadvantages.

According to the present invention there is provided a tool for use in removing plastics material from a hole comprising a work surface having a substantially annular cutting edge adapted to contact said material, piezo electric transducer means operatively connected through a work horn to said work surface to cause it to vibrate ultrasonically and thereby to heat locally said plastics material, cavity means adapted to receive said heated plastics material, means to connect said cavity means to a working zone adjacent said work surface, wherein the work surface includes at least one cutting fin extending radially outwardly of the annular cutting edge.

Preferably four cutting fins are provided, disposed substantially equiangularly around the periphery of the annular cutting edge of the work surface.

The cutting fins are particularly useful for removing cement at the proximal end of the femur. The fins cut grooves in the cement almost to the endosteal surface of the femur, allowing the cement subsequently to be removed in pieces with ease.

Preferably, at least the piezo electric transducer means is sealingly encased in a first enclosure of waterproof plastics material and exterior thereof a second enclosure of stainless steel or the like material.

Advantageously, the waterproof plastics material is an acetal plastics material.

This arrangement allows the tool to be autoclaved or otherwise sterilised for use for another patient.

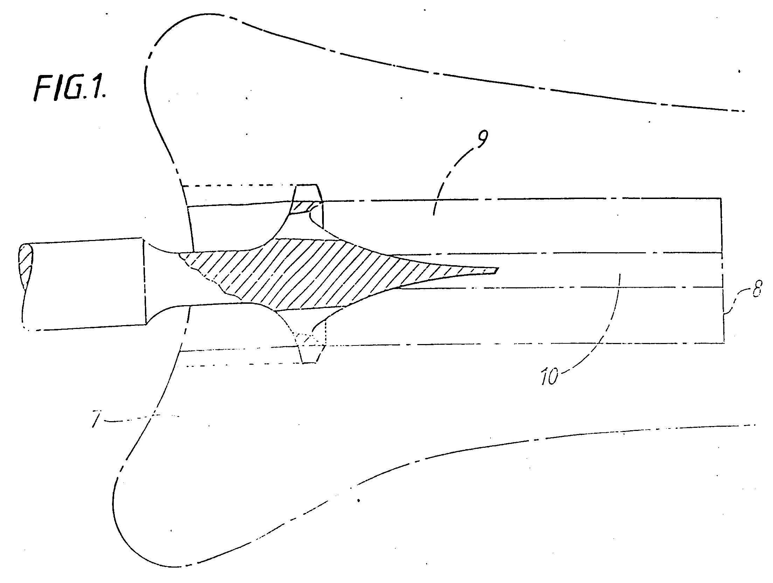

Embodiments of the present invention will now be more particularly described by way of example and with reference to the accompanying drawings, in which: -
FIGURE 1 is a schematic side elevation, shown partially in cross-section, of a tool embodying a first aspect of the invention;

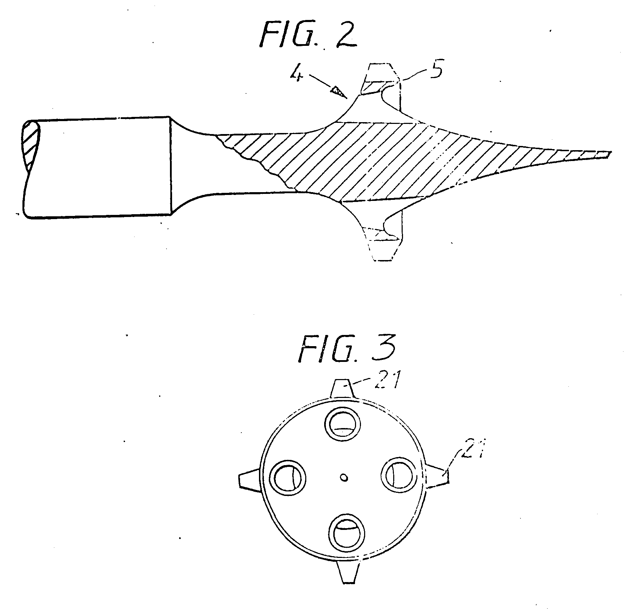

FIGURE 2 is a schematic side elevation, partially in cross section, of an end portion of a tool of the invention;
FIGURE 3 is an end elevation of the tool end portion of Figure 2;
FIGURE 4 shows in cross section a preferred version of the invention; and
FIGURE 5 is a cross section of a further embodiment of handle for use with the invention.

Referring now to the drawings, there is shown in the Figures a tool comprising a piezo electric ceramic transducer 1, connected along a longitudinal axis to a coupling horn 2, which in turn is connected along the longitudinal axis to a work horn 3. At the far end of the work horn 3 is a cavity 4 surrounded by an annular cutting edge 5.

As shown in the Figures, the length of the piezo electric ceramic transducer 1 is half a wavelength, the length of the coupling horn 2 is a full wavelength, while the length of the work horn 3 (which includes the annular cutting edge 5) is an integral number of half wavelengths ensuring that the total probe length can penetrate to the required depth. The term "wavelength" is used to represent the wavelength of the ultrasonic wave generated by the piezo electric ceramic transducer in the material concerned. The preferred material for the work horn and annular cutting edge is titanium or an alloy thereof. At an ultrasonic vibrational frequency in the region of 30-35 kHz, the wavelength of the ultrasonic wave in the titanium alloy is in the region of 70-90mm.

It is well known that many common plastics materials will transmit high frequency vibrations without the significant internal losses which would cause bulk heating of the material. It is also known that when the ultrasound is transmitted through two closely contacted components, the interface can experience a considerable heating effect which under the correct circumstances will produce welding. Such heating can also occur at the interface between a vibrating tool or metal component and the plastics material, the heating being sufficient to melt the plastic. The present invention utilises this effect to drill an annular hole into the plastics material.

The plastics cement material used for hip joint replacements is generally a powder of polymethylmethacrylate beads of diameter less than 100 µm held together in situ by a polymerised methyl methacrylate monomer. This material is prone to creep and is susceptible to localised heating on ultrasonic vibration. The property of creep may be utilised in that, during removal of a core of plastics cement material, the existing cement which remains may be forced into improved engagement with fissures or surface imperfections in the bone by virtue of the ultrasonic vibrations imparted to the cement, and thereby stabilise the interface.

At the work surface, the annular cutting edge can be manipulated by the user of the tool to enable the bore diameter to be widened or, by applying pressure to one side of the tool, to create a hole of oval profile.

The present invention is described with reference to removing a plastics cement from a hip joint replacement during revision of the prosthesis. In this case, the hip bone or femur 7 has a blind hole 8 filled with plastics cement 9 which had originally surrounded the prosthesis, but which has a void 10 where the prosthesis used to be.

In order to operate the tool, the tip is inserted a short distance into the plastics material cement 9 and pushed thereinto for about 5-10mm, as the plastics material softens under the effect of the ultrasonic vibrations. At this point, a core of softened but relatively stiff cement fills the work horn cavity 4.

The sequence is then repeated until the cement is removed from the bore to an appropriate depth. It would be possible to incorporate a small intrascope coaxially within an axial duct in order to facilitate visual inspection of the cutting operation.

Use of the tool embodying the present invention results in a much faster cutting operation and also allows the possibility of leaving intact a thin layer of cement which is characteristically well-bonded to the living bone tissue when revising damaged but not loose implants. If the cement is well-bonded, the strength of the revised implants would be significantly improved. The apparatus also may permit improved bonding between bone and existing cement.

Whereas the existing methods of revision of hip joint prosthesis may have required several hours to remove the existing cement, for all of which the patient must be anaesthetized, the present invention allows removal of existing cement, at least sufficient for revision, within a period of less than one hour. The work horn 3 may be curved to suit penetrating of a curved hole in a medulla or similar bone.

For use at or adjacent the proximal end of the femur, it may be necessary to remove a core of cement of larger diameter. In this case, as shown in the Figures, four external fins 21 are provided to cut grooves in this thicker cement. Once the tool has been withdrawn, the pieces of cement remaining between the grooves may be removed with ease.

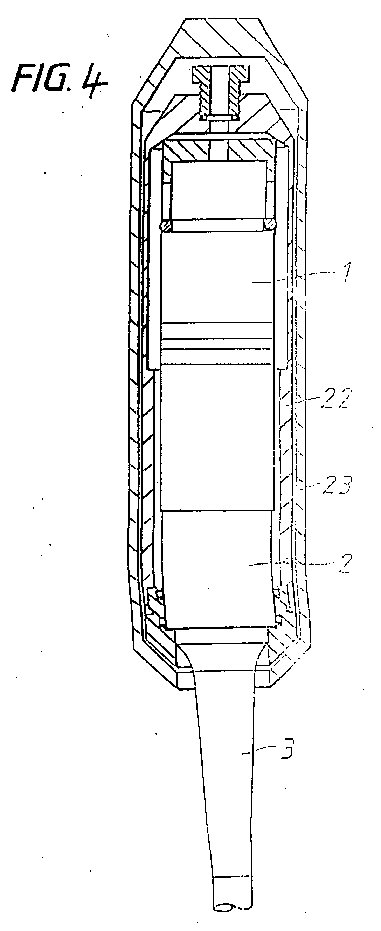

As shown in Figure 4, the piezo electric transducer part of the tool may be encased, first in a layer of acetal plastics material 22 and then in a layer 23 of stainless steel. This arrangement will allow the tool to be autoclaved or otherwise sterilised in order to permit its use on further patients.

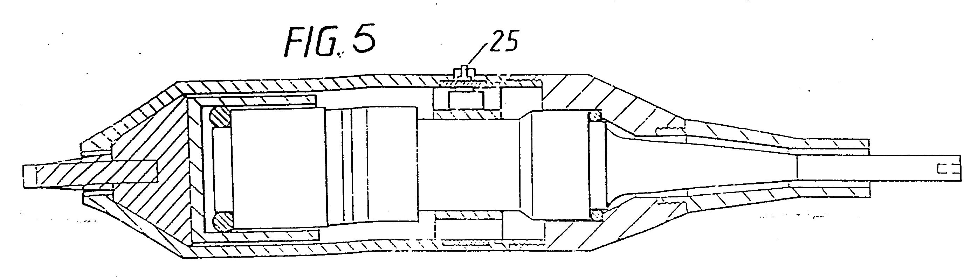

As shown in Figure 5, the handset part of the tool may incorporate a switch 25 and still be autoclavable.

The design philosophy takes account of the conditions prevailing in operating theatres and in particular the strict sterilisation requirements. Several sizes and shapes of oscillatory instrument should ideally be available to the surgeon and a particular case might demand very specialised probe designs. Whereas it is possible to interchange probes on a single handset this procedure is not only inconvenient but results in reduced efficiency of the system if the critical probe/horn interface becomes contaminated with foreign matter. It is desirable therefore to provide a number of independent handsets which can be selected by the surgeon without the need for reconnection or adjustment of switching functions. This dictates the use of a switch incorporated in each handset. Since the handset and cable assembly must be suitable for autoclave sterilisation, the switch assembly requires a special seal design to withstand the temperature and pressure conditions encountered during the sterilisation process.

Figure 5 shows a handset which includes a coaxial switch button operating a sub-miniature micro-switch via a cylindrical moulded seal. The switch is contained in a metal cylindrical sleeve which supports the seal and ensures that it remains water tight even under pressure. This design permits the construction of an oscillatory system offering maximum operating flexibility with inherent reliability. Furthermore there is no need for foot switches which for up to four handsets would involve impractical complications.

## Claims

1. A tool for use in removing plastics material (9) from a hole (8) comprising a work surface having a substantially annular cutting edge (5) adapted to contact said material, piezo electric transducer means (1) operatively connected through a work horn (3) to said work surface to cause it to vibrate ultrasonically and thereby to heat locally said plastics material, cavity means (4) adapted to receive said heated plastics material, means to connect said cavity means to a working zone adjacent said work surface, characterised in that the work surface includes at least one cutting fin (21) extending radially outwardly of the annular cutting edge (5) of the work surface.

2. A tool as claimed in Claim 1, characterised in that four cutting fins (21) are provided, disposed substantially equiangularly around the periphery of the annular cutting edge (5).

3. A tool as claimed in either Claim 1 or Claim 2 characterised in that the fins are adapted to cut grooves in the cement which extend almost to the endosteal surface of a femur or other bone structure, allowing the cement subsequently to be removed in pieces with ease.

4. A tool as claimed in any one of the preceding claims, characterised in that at least the piezo electric transducer means is sealingly encased in a first enclosure of water proof plastics material (22) and exterior thereof a second enclosure of stainless steel (23) or the like material.

5. A tool as claimed in Claim 4, characterised in that the waterproof plastics material (22) is an acetal plastics material.

6. A tool as claimed in either Claim 4 or Claim 5, characterised in that switch means (25) are provided on a handset of said tool, said switch means (25) being sealed to allow the tool to be autoclaved or otherwise sterilised for use for another patient.

## Patentansprüche

1. Werkzeug zum Entfernen von Kunststoffmaterial (9) aus einer Bohrung (8), umfassend eine Arbeitsfläche mit einer im wesentlichen ringförmigen, zum Kontakt mit dem Material geeigneten Schneidkante (5), eine piezoelektrische Wandlereinheit (1), die über ein Arbeitshorn (3) wirkungsmäßig mit der Arbeitsfläche verbunden ist, um diese in Ultraschallschwingungen zu versetzen und damit das Kunststoffmaterial örtlich zu erhitzen, ein für die Aufnahme des erhitzten Kunststoffmaterials geeignetes Hohlraummittel (4) sowie Mittel zum Verbinden des Hohlraummittels mit einer Arbeitszone an bzw. neben der Arbeitsfläche, dadurch gekennzeichnet, daß die Arbeitsfläche mindestens eine(n) von der ringförmigen Schneidkante (5) der Arbeitsfläche radial nach außen ragende(n) Schneidrippe bzw. Schneidgrat (21) aufweist.

2. Werkzeug nach Anspruch 1, dadurch gekennzeichnet, daß vier Schneidrippen bzw. -grate (21) vorgesehen sind, die um den Umfang der ringförmigen Schneidkante (5) in im wesentlichen gleichem Winkelabstand angeordnet sind.

3. Werkzeug nach Anspruch 1 oder 2, dadurch gekennzeichnet, daß die Schneidrippen bzw. -grate geeignet sind, Rillen in den Zement zu schneiden, die beinahe bis zur endostealen Oberfläche eines Femurs oder einer anderen Knochenstruktur reichen, was ein nachfolgendes leichtes Entfernen des Zements in Stücken gestattet.

4. Werkzeug nach einem der vorangehenden Ansprüche, dadurch gekennzeichnet, daß mindestens die piezoelektrische Wandlereinheit abgedichtet in einer ersten Ummantelung (22) aus wasserdichtem Kunststoffmaterial und einer an dessen Außenseite vorgesehenen zweiten Ummantelung (23) aus nichtrostendem Stahl oder dgl. eingekapselt ist.

5. Werkzeug nach Anspruch 4, dadurch gekennzeichnet, daß das wasserdichte Kunststoffmaterial (22) ein Acetalkunststoff ist.

6. Werkzeug nach Anspruch 4 oder 5, dadurch gekennzeichnet, daß an einem Handapparat des Werkzeugs Schaltermittel (25) vorgesehen sind, welche Schaltermittel (25) abgedichtet sind, um eine Autoklavenbehandlung oder anderweitige Sterilisierung des Werkzeugs für Verwendung bei einem anderen Patienten zu gestatten.

## Revendications

1. Instrument destiné à être utilisé pour enlever de la matière plastique (9) d'un trou (8), qui comprend une surface de travail comportant un bord de coupe sensiblement en anneau (5) prévu pour entrer en contact avec ladite matière plastique, un moyen transducteur piézo-électrique (1) relié par un pavillon actif (3) à ladite surface de travail pour la faire vibrer à une fréquence ultrasonore et ainsi chauffer localement ladite matière plastique, un moyen formant cavité (4) prévu pour recevoir ladite matière plastique chauffée, un moyen pour relier ledit moyen formant cavité à une zone de travail voisine de ladite surface de travail, caractérisé en ce que la surface de travail comprend au moins une nervure coupante (21) s'étendant radialement vers l'extérieur du bord coupant en anneau (5) de la surface de travail.

2. Instrument selon la revendication 1, caractérisé en ce que quatre nervures coupantes (21) sont fournies, qui sont disposées sensiblement à des distances angulaires également réparties autour de la périphérie du bord de coupe en (5).

3. Instrument selon la revendication 1 ou 2 caractérisé en ce que les nervures sont prévues pour couper des rainures dans le ciment qui s'étend presque jusqu'à la surface endostale d'un fémur ou d'une autre structure osseuse, ce qui permet d'enlever ensuite le ciment par morceaux avec facilité.

4. Instrument selon l'une quelconque des revendications précédentes, caractérisé en ce que qu'au moins le moyen transducteur piézo-électrique est logé de façon étanche dans un premier boîtier en matière plastique étanche à l'eau (22) à l'extérieur duquel on trouve un second boîtier (23) en acier inoxydable ou en un matériau similaire.

5. Instrument selon la revendication 4, caractérisé en ce que la matière plastique étanche à l'eau (22) est une matière plastique acétalique.

6. Instrument selon la revendication 4 ou 5, caractérisé en ce que des moyens de commutation (25) sont disposés sur une poignée dudit instrument, lesdits moyens de commutation (25) étant fermés hermétiquement pour pouvoir passer l'instrument à l'autoclave ou le stériliser par un autre moyen afin de l'utiliser pour un autre patient.
